Europäisches Patentamt

European Patent Office

Office européen des brevets

(19)

(11) Numéro de publication : **0 285 525 B1**

(12) **FASCICULE DE BREVET EUROPEEN**

(45) Date de publication du fascicule du brevet :
**15.01.92 Bulletin 92/03**

(51) Int. Cl.⁵ : **C08L 83/04,** A61K 9/22,
C08K 13/02, A61K 33/18,
C02F 1/76

(21) Numéro de dépôt : **88420062.7**

(22) Date de dépôt : **23.02.88**

(54) **Composition à base de gomme diorganopolysiloxane contenant del'iode pour le traitement des eaux.**

(30) Priorité : **26.02.87 FR 8702882**

(43) Date de publication de la demande :
**05.10.88 Bulletin 88/40**

(45) Mention de la délivrance du brevet :
**15.01.92 Bulletin 92/03**

(84) Etats contractants désignés :
**AT BE CH DE ES FR GB GR IT LI LU NL SE**

(56) Documents cités :
DE-A- 1 467 861
GB-A- 1 412 970
US-A- 2 918 400
US-A- 4 275 194
US-A- 4 384 960
US-A- 4 420 590
US-A- 4 640 956

(72) Inventeur : **Cyprien, Guy**
**7, rue d'Anjou**
**F-94240 l'Hay Les Roses (FR)**
Inventeur : **Fisch, Alain**
**22, rue de l'Yvette**
**F-75016 Paris (FR)**
Inventeur : **Haggiage, Johnny**
**72, rue du Fort Saint-Irénée**
**F-69005 Lyon (FR)**
Inventeur : **Porte, Hugues**
**6, chemin de Crépieux**
**F-69300 Caluire (FR)**
Inventeur : **Prazuck, Thierry**
**53, avenue Mathurin Moreau**
**75019 Paris (FR)**
Inventeur : **Torres, Ghislaine**
**104, rue Ney**
**69006 Lyon (FR)**

(74) Mandataire : **Fabre, Madeleine-France et al**
**RHONE-POULENC CHIMIE Direction des**
**Brevets Secteur Spécialités Chimiques 25,**
**quai Paul-Doumer**
**F-92408 Courbevoie Cédex (FR)**

(73) Titulaire : **RHONE-POULENC CHIMIE**
**25, quai Paul Doumer**
**F-92408 Courbevoie Cédex (FR)**

Jouve, 18, rue Saint-Denis, 75001 PARIS

EP 0 285 525 B1

## Description

La présente invention concerne une composition de silicone à base de gomme diorganopolysiloxane contenant de l'iode ainsi qu'un procédé de traitement des eaux d'usage domestique et de boissons à l'aide de cette composition.

On estime actuellement à plusieurs centaines de millions le nombre de sujets présentant une déficience ou une carence en iode dans le monde. Les zones géographiques les plus touchées sont l'Amérique Latine, en particulier le long de la Cordillière des Andes, la quasi totalité des pays non côtiers de l'Afrique et de l'Asie (Pakistan, Inde, Népal, Chine, Laos, etc.....).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les deux principales actions menées jusqu'àlors sont demeurées inéfficaces.

Ces actions visent essentiellement d'une part :

— l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajouté au sel s'en échappe rapidement lorsqu'il n'est pas parfaitement emballé.

et d'autre part :

— l'injection intra-musculaire d'huile iodée : cette injection a l'avantage de présenter une action différée (action retard) mais elle n'est pas sans présenter des inconvénients, en particulier les risques d'infection, les risques d'allergie à l'iode, les risques d'hyperthyroïdie ou d'hypothyroïdie induites par l'injection d'une dose nécessairement supra-physiologique.

On a par ailleurs décrit dans le brevet belge BE-A-889680 l'introduction d'oligo-éléments, dont l'iode, dans l'eau de boisson des ruminants, sous la forme d'une dispersion dans un liant comme par exemple du plâtre de Paris. Un diorganopolysiloxane peut être ajouté en vue de retarder la diffusion de l'oligo-élément. En outre l'utilisation d'iode et de composés de l'iode pour désinfecter ou pour purifier l'eau est bien connue. On peut citer à titre d'exemple les brevets américains US-A2347567, US-A-2743208 et US-A-3408295.

Il existe également de très nombreux brevets décrivant l'utilisation de systèmes polymères, en particulier de silicone, pour la libération contrôlée d'un principe actif au moyen par exemple d'un système transdermique (brevet américain US-A-4053580), ou par absorption buccale notamment pour des ruminants (brevet français FR-A-2560768).

Enfin par le brevet américain US-A-4384960 il est décrit la mise en pastilles d'iode $I_2$ dans une bouteille en plastique dans laquelle l'eau pénètre par une membrane poreuse de polymère. L'eau solubilise l'iode. Le rôle de la membrane est seulement d'éviter que l'iode en pastilles ne sorte de la bouteille.

Il est simplement suggéré en outre qu'il est possible d'introduire l'iode $I_2$ dans la bouteille au sein d'une dispersion liquide de silicone ou d'un élastomère de diméthylsiloxane puis de les faire durcir. Cette solution indiquée n'est pas techniquement réalisable car d'une part $I_2$ est un inhibiteur bien connu des catalyseurs de durcissement des élastomères silicones vulcanisables à température ambiante (voir en particulier la publication de W.D. MORAIN et al. Plastic and Reconstructive Surgery 59, 2, 215-222 (1977)), et d'autre part du fait de sa volatilité élevée, $I_2$ se sublime lors de la réticulation à chaud des élastomères silicones.

Toutefois dans ce système il n'y a pas de contrôle de la libération de l'iode d'une part, et, d'autre part, l'iodation de l'eau se fait par addition discontinue ou en continu de quelques gouttes d'eau très iodée (à saturation) contenue dans la bouteille, dans un récipient quelconque contenant de l'eau non traitée. Il est clair que la solution proposée par US-A-4384960 est imparfaite, par le fait notamment qu'il s'agit d'une technique individuelle qui, comme l'injection intra-musculaire d'iode, nécessite une éducation et une mobilisation massive des populations.

Le brevet US-A-4420590 enseigne que de l'eau peut être rendue potable après passage au contact d'une résine échangeuse d'anion traitée par essentiellement :

— de l'iode élémentaire,

— du bromure de potassium,

— de l'iodure de potassium,

dans des proportions relatives bien définies

Le brevet US-A-4384960 enseigne qu'il est possible de prévenir les conséquences d'une carence en iode

2

en ajoutant de l'iode élémentaire (c'est-à-dire au degré d'oxydation zéro) à de l'eau.

La demande allemande DE-A-1467861 enseigne que des médicaments, tels que par exemple des hormones, vitamines, antibiotiques, anticoagulants, peuvent être introduits dans l'organisme alors qu'ils se trouvent dans des élastomères silicones.

La présente invention a précisément pour but de proposer une composition de silicone contenant de l'iode utilisable pour le traitement continu des eaux d'usage domestique, en particulier dans les systèmes d'adduction et de traitement des eaux dans les puits et les forages et qui permette de relarguer (libérer) une quantité contrôlée et adaptée d'iode en vue d'assurer d'une part le traitement collectif des diverses manifestations dues à une carence en iode et d'autre part une prophylaxie de ces diverses manifestations.

Elle a également pour but de proposer une composition de silicone contenant de l'iode qui immergée de façon appropriée dans les endroits contenant l'eau à traiter, en particulier les puits et forages, relargue (libère) de façon continue, de préférence pendant au moins un an, une quantité appropriée d'iode sous une forme et à une dose thérapeutiquement active et efficace pour soigner les diverses maladies dues à une carence en iode.

Elle a également pour but de proposer une composition de silicone contenant de l'iode qui, immergée de façon appropriée dans les endroits contenant l'eau à traiter n'ait aucune action secondaire indésirable et néfaste sur le plan chimique et biologique sur les eaux à traiter.

Elle a également pour but de proposer une composition de silicone contenant de l'iode présentant une forme adaptée au milieu dans lequel se trouve l'eau à traiter, cette forme étant adaptée en particulier aux puits et/ou forages et présentant un système d'introduction dans les puits et/ou forages permettant son remplacement aisé.

Ces buts et d'autres sont atteints par la présente invention qui concerne en effet une composition de silicone comportant :

(A) une gomme diorganopolysiloxane dont la viscosité à 25°C est supérieure à 1000000 mPa · s,

($B_2$) un péroxyde organique ($B_2$),

($B_1$) éventuellement une charge renforçante,

(C) au moins un composé de l'iode à l'exception de l'iode moléculaire $I_2$, solide à la température ambiante, soluble dans l'eau, non toxique, dispersé de façon homogène dans la composition.

Comme composé de l'iode, à l'exception de l'iode moléculaire $I_2$, on peut utiliser seul ou en mélange :

— les iodures ou iodates de formules générales :

$$(M^{a+}) (I^-)_a$$
$$\text{et} \quad (M^{a+}) (IO_3{}^-)_a$$

dans lesquelles a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NY_4)^+$, dont les radicaux Y identiques ou différents représentent un radical alkyle linéaire ou ramifié en $C_1$-$C_{20}$ ou un atome d'hydrogène, tel que l'ion ammonium $NH^+{}_4$.

Les cations $M^{a+}$ et $NY_4{}^+$, sont choisis de telle sorte que l'iodure ou l'iodate correspondant soit un solide à température ambiante, soit soluble dans l'eau et soit non toxique.

Comme iodures et iodates on peut en particulier utiliser ceux de formules :

NaI, NaIO$_3$,

KI, KIO$_3$,

MgI$_2$, MgI$_2$ · 8H$_2$O,

Mg(IO$_3$)$_2$ · 4H$_2$O,

NH$_4$I

FeI$_2$ · 4H$_2$O

MnI$_2$.

Ces sels peuvent contenir de l'eau d'hydratation ou de l'eau de constitution.

Pour des raisons de facilité de mise en oeuvre les composés d'iode solides sont préférés, et parmi ceux-ci NaI et KIO$_3$ sont les plus préférés.

Tous les composés de l'iode tels que définis ci-dessus, lorsqu'ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode soluble dans l'eau on entend un composé présentant une solubilité d'au moins 100 µg/l à température ambiante.

On utilise généralement de 5 à 150 parties de préférence de 10 à 100 parties de composé de l'iode (C) pour 100 parties de gomme (A).

En particulier dans les pays en développement, l'eau à usage domestique (boisson, lavage, irrigation, etc.....) est pour l'essentiel apportée par deux types de structure, les puits et les forages.

Pour des raisons évidentes de coût, d'efficacité, de salubrité, la création nouvelle d'un point d'eau se fait souvent par forage.

Un forage est une colonne d'air forée à travers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'une colonne de 10 à 70 mètres, généralement de 30 à 50 mètres de haut qui est extraite à l'aide d'une pompe à corps immergé.

Cette eau se renouvelle principalement en fonction de l'utilisation du forage qui dépend de la saison. En effet en saison des pluies le forage est traditionnellement moins utilisé. Par contre en saison sèche, le forage débite environ 10-12 heures par jour, soit une quantité comprise entre 5 et 10 m$^3$ par jour pendant environ six mois.

En règle générale, un puits peut être asséché deux fois durant la journée au moment de la saison sèche ce qui correspond avec ces données statistiques moyennes, à un maximum d'utilisation de 5 à 10 m$^3$.

De nombreuses études montrent que dans les zones de grande endémie goîtreuse, le taux pré-existant d'équivalent en iode dans l'eau des forages ou des puits est inférieur à 2 microgrammes par litre (2 μg/l). Il est estimé actuellement qu'un apport journalier d'envrion 100 μg d'équivalent iode par jour et par personne serait suffisant pour prévenir le développement d'un goître endémique et sans doute environ 150 μg lorsqu'il y a consommation régulière de substances goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 μg de préférence environ 100 μg, d'équivalent iode par jour constitue le but recherché.

Ainsi sachant qu'un individu adulte absorbe en moyenne 2 litres d'eau par jour et sur la base des données ci-dessus (forage ayant un débit de 600 l/h), il apparaît souhaitable qu'un litre d'eau traitée contienne environ 50 μg/l d'iode ce qui correspond à 50 μg d'équivalent iode par litre et par personne, ce qui nécessite que la composition de silicone relargue 720 mg/j d'équivalent d'iode, soit 270 g d'équivalent iode à relarguer pendant une année.

Dans ce qui suit ou ce qui précède, sauf indications contraires, les parties et pourcentages sont en poids.

De façon surprenante et inattendue on a en effet découvert selon la présente invention qu'il est possible d'ajouter dans une gomme silicone des quantités importantes de composé d'iode sous une forme solide ou liquide tel que défini ci-dessus, à savoir p.ex. de 5 à 150 parties, de préférence de 20 à 100 pour 100 parties de gomme diorganopolysiloxane préalablement chargée avec une charge renforçante et d'obtenir ainsi un produit qui présente des caractéristiques mécaniques suffisantes pour l'application envisagée et qui, immergé dans l'eau permette d'assurer un relargage continu et contrôlé d'iode de préférence pendant au moins un an.

Le système de libération contrôlée de l'iode fait partie des systèmes matriciels dont la diffusion du principe actif est normalement régie par la loi de FICK, c'est-à-dire par une cinétique de diffusion d'ordre 1/2 pour seulement 60% en poids du principe actif. Au-delà de 60% il y a épuisement de la matrice et les flux de diffusion sont fortement diminués. De façon surprenante et inattendue, on a trouvé que le système matriciel silicone conforme à l'invention libère l'iode suivant une cinétique d'ordre 0 et de façon continue et cela jusqu'à ce que 80% en poids et plus du composé d'iode soit libéré.

L'avantage considérable apporté par la matrice silicone est donc qu'il est très facile d'extrapoler la diffusion continue du principe actif après une mesure de la quantité libérée au bout d'au moins un mois puisque l'on sait que la cinétique de diffusion est d'ordre 0 et qu'au moins 80% du composé d'iode sera libéré suivant cette cinétique.

De façon à maîtriser la libération du principe actif, il est avantageux de présenter la matrice silicone sous la forme de modules (éléments) élémentaires de formes variées tels que des cubes, des parallélépipèdes rectangles, des cylindres, des sphères dont les paramètres fondamentaux sont les suivants :
— la nature du composé d'iode,
— le diamètre moyen (granulométrie) g des particules du composé d'iode dans dans le cas préféré où ce dernier est un solide,
— la teneur de composé d'iode au sein de la matrice,
— le rapport R de la surface au volume du module.

La nature du composé d'iode et sa granulométrie définissent la vitesse de diffusion à travers la matrice, du principe actif.

Plus g est petit et plus v est lent et inversement.

Plus t est grand et plus le flux de principe actif est élevé et inversement.

Plus R est grand et plus le flux élevé de principe actif est grand et inversement.

L'homme de métier, par des expériences de routine, peut sans difficulté aboutir rapidement au résultat visé en extrapolant le temps d'élution théorique qui correspondra au temps réel de diffusion du principe actif.

Pour NaI et KIO$_3$ qui sont les composés d'iode préférés, g, t et R peuvent être avantageusement choisis dans les zones suivantes :

— g compris entre 1 et 300 μm,

— t compris entre 10 et 100 parties en poids de composé d'iode pour 100 parties de (A),

— R compris entre 0,5 et 50 pour une forme cylindrique.

Il est en outre souhaitable que le composé d'iode soit dispersé de façon homogène au sein de la matrice.

Plus précisément la présente invention concerne une composition de silicone comportant :

— (A) : 100 parties d'une gomme diorganopolysiloxane ayant une viscosité supérieure à 1 Million mPa · s à 25°C,

— (B) : 5 à 130 parties d'une charge renforçante (B$_1$), de préférence siliceuse, choisie parmi les silices de pyrogénation et les silices de précipitation, et/ou de 0,1 à 6 parties d'un péroxyde organique (B$_2$),

— (C) : 5 à 150 parties d'un composé de l'iode à l'exception de l'iode moléculaire I$_2$, solide à la température ambiante, soluble dans l'eau, non toxique, dispersé de façon homogène dans la composition.

Plus particulièrement la gomme diorganopolysiloxane (A) a pour formule générale R$_{3-a}$(R'O)$_a$SiO(R$_2$SiO)$_n$Si(OR')$_a$R$_{3-a}$ dans laquelle les symboles R, identiques ou différents, représentent des radicaux hydrocarbonés en C$_1$-C$_8$, substitués ou non par des atomes d'halogène, des radicaux cyano ; le symbole R' représente un atome d'hydrogène, un radical alkyle en C$_1$-C$_4$, le symbole a représente zéro ou un, le symbole n représente un nombre ayant une valeur suffisante pour obtenir une viscosité d'au moins 1 Million de mPa·s à 25°C, au moins 50% en nombre de radicaux représentés par R sont des radicaux méthyle.

De préférence 0,005 à 0,5 mole % des motifs entrant dans la constitution de la gomme (A) sont choisis parmi ceux de formules (CH$_2$=CH)(R)SiO et/ou (CH$_2$=CH)R$_{2-a}$(RO')$_a$Si$_{0,5}$.

La gomme (A) de viscosité de 1 Million de mPa · s à 25°C, de préférence d'au moins 2 Millions de mPa·s à 25°C, est constituée, le long de sa chaîne, de motifs R$_2$SiO et elle est bloquée à chaque extrémité de sa chaîne par un motif R$_{3-a}$R(O)$_a$SiO$_{0,5}$ ; cependant la présence en mélange avec ces motifs, de motifs de structure différente, par exemple de formule RSiO$_{1,5}$ et SiO$_2$, n'est pas exclue dans la proportion d'au plus 2% par rapport au nombre total des motifs R$_2$SiO et R$_{3-a}$(RO')$_a$SiO$_{0,5}$.

Le symbole R représente un radical hydrocarboné en C$_1$-C$_8$ substitué ou non par des atomes d'halogène, des radicaux cyano ; il englobe plus spécifiquement :

— des radicaux alkyle en C$_1$-C$_5$, substitués ou non par des atomes d'halogène, des radicaux cyano, tels que les radicaux méthyle, éthyle, propyle, isopropyle, butyle, isobutyle, pentyle, trifluoro-3,3,3 propyle, β-cyanoéthyle, γ-cyanopropyle,

— des radicaux alcényle en C$_2$-C$_4$ tels que les radicaux vinyle, allyle, butène-2 yle,

— des radicaux aryle mononucléaire en C$_6$-C$_8$, substitués ou non par des atomes d'halogène tels que les radicaux phényle, chlorophényle, tolyle, trifluorométhylphényle.

Les radicaux alkyle en C$_1$-C$_4$, représentés par le symbole R', concernent plus spécifiquement les radicaux méthyle, éthyle, propyle, isopropyle, butyle, butyle secondaire.

Au moins 50% en nombre, de préférence au moins 70% des radicaux représentés par R sont des radicaux méthyle.

Par ailleurs des radicaux vinyle sont de préférence également présents, en quantité appropriée, dans la gomme (A) ; ils conduisent à des motifs de formule CH$_2$=CH(R)SiO et CH$_2$=CH(R$_{2-a}$)(R'O)$_a$SiO$_{0,5}$ dont le nombre représente 0,005 à 0,5 mole %, de préférence 0,01 à 0,45 mole %, de l'ensemble des motifs de formules générales R$_2$SiO et R$_{3-a}$(R'O)$_a$SiO$_{0,5}$, entrant dans la constitution de la gomme (A).

A titre d'exemples concrets de motifs constituant les gommes (A) peuvent peuvent être cités ceux de formules :

(CH$_3$)$_2$SiO,
CH$_3$(CH$_2$=CH)SiO,
CH$_3$(C$_6$H$_5$)SiO,
(C$_6$H$_5$)$_2$SiO,
CH$_3$(C$_2$H$_5$)SiO,
CH$_3$CH$_2$-CH$_2$(CH$_3$)SiO,

$CH_3(n \cdot C_3H_7)SiO,$

$(CH_3)_3SiO_{0,5},$

$(CH_3)_2CH_2=CHSiO_{0,5},$

$CH_3(C_6H_5)_2SiO_{0,5},$

$CH_3(C_6H_5)(CH_2=CH)SiO_{0,5},$

$HO(CH_3)_2SiO_{0,5},$

$CH_3O(CH_3)_2SiO_{0,5},$

$C_2H_5O(CH_3)_2SiO_{0,5},$

$n \cdot C_3H_7O(CH_3)_2SiO_{0,5},$

$HO \cdot (CH_2=CH)(CH_3)SiO_{0,5}.$

Les gommes (A) sont commercialisées par les fabricants de silicones, d'autre part elles peuvent être aisément fabriquées en mettant en oeuvre des techniques abondamment décrites dans la littérature chimique.

Dans la majorité des cas, on utilise des gommes méthylvinyldiméthylpolysiloxanes possédant le long de leur chaîne des motifs $(CH_3)_2SiO$ et $CH_2=CH(CH_3)SiO$ et à l'extrémité de leur chaîne des motifs choisis parmi ceux de formules :

$(CH_3)_2CH_2=CHSiO_{0,5},$ $HO(CH_3)(CH_2=CH)SiO_{0,5},$

$(CH_3)_3SiO_{0,5},$ $C_6H_5(CH_3)CH_2=CHSiO_{0,5},$

$HO(CH_3)_2SiO_{0,5},$

ou des gommes diméthylpolysiloxanes bloquées à chaque extrémité de leur chaîne par l'un des motifs précédents renfermant un radical vinyle.

Elles présentent généralement une viscosité d'au moins 2 Millions de mPa $\cdot$ s à 25°C.

Les charges (B$_1$) qui sont de préférence des silices renforçantes sont utilisées à raison de 5 à 130 parties des gommes diorganopolysiloxanes (A). Elles sont choisies parmi les silices de combustion et les silices de précipitation. Elles ont une surface spécifique, mesurée selon les méthodes BET, d'au moins 50 m²/g, de préférence supérieure à 70 m²/g, une dimension moyenne des particules primaires inférieure à 0,1 µm (micromètre) et une densité apparente inférieure à 200 g/litre.

Ces silices peuvent être incorporées telles quelles ou après avoir été traitées par des composés organosiliciques habituellement utilisés pour cet usage. Parmi ces composés, figurent les méthylpolysiloxanes tels que l'hexaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthyldichlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane. Lors de ce traitement, les silices peuvent accroître leur poids de départ jusqu'à un taux de 20%, de préférence 18% environ.

Les compositions selon l'invention peuvent être malaxées à froid telles quelles et être extrudées par exemple sous la forme d'un cylindre de diamètre compris entre 0,5 et 9 cm. Les cylindres de composition de silicone obtenues peuvent être découpées dans le cas de leur utilisation dans les forages à la longueur désirée de manière à ce que le cylindre comporte une quantité suffisante en équivalent iode pour un relargage pendant une année.

Les péroxydes organiques (B$_2$) sont utilisés à raison de 0,1 à 6 parties, de préférence 0,2 à 5 parties, pour 100 parties des gommes (A). Ils sont bien connus des techniciens et comprennent plus spécialement le péroxyde de benzoyle, le péroxyde de dichloro-2,4 benzoyle, le péroxyde de dicumyle, le bis(t-butylpéroxy)2,5 diméthyl-2,5 hexane, le perbenzoate de t-butyle, le carbonate de péroxy t-butyle et d'isopropyle, le péroxyde de di-t-butyle, le bis(t-butylpéroxy)-1,1 triméthyl-3,3,5 cyclohexane.

Ces divers péroxydes se décomposent à des températures et à des vitesses parfois différentes. Ils sont choisis en fonction des conditions de durcissement exigées.

Les compositions de silicone selon l'invention peuvent comporter en outre, pour 100 parties de gomme (A) de 0,1 à 6 parties d'un agent de texturation (D) qui est un polymère organofluoré sous forme d'un solide pulvérulent.

Les polymères fluorés (D) sont utilisés à raison de 0,1 à 6 parties, de préférence 0,15 à 5 parties, pour 100 parties des gommes diorganopolysiloxaniques (A). Ces composés sont bien connus des techniciens ; ils sont préparés par polymérisation ou copolymérisation de monomères choisis par exemple dans le groupe du tétrafluoroéthylène, du chlorotrifluoroéthylène, du fluorure de vinylidène, de l'hexafluoropropène. Ce sont donc des polymères ou des copolymères constitués de motifs dérivés des monomères précédents ; ainsi sont utilisables des polytétrafluoroéthylènes, les copolymères binaires du type polytétrafluoroéthylène-béta-fluoropropène, ou du type fluorure de vinylidène-hexafluoropropène, les copolymères ternaires du type fluorure de vinylidène-hexafluoropropène-tétrafluoroéthylène.

Ces composés peuvent être introduits dans les compositions de l'invention sous la forme de poudres de diamètre particulaire moyen inférieur à 100 micromètres, par exemple de diamètre allant de 25 à 65 micromètres.

On peut remplacer jusqu'à 90% en poids des silices de renforcement (B$_1$) par des charges semi-renforçantes ou de bourrage dont le diamètre particulaire est supérieur à 0,1 µm, telles que le quartz broyé, les argiles calcinées et les terres de diatomées.

Les compositions de silicone peuvent comporter en outre de 1 à 10 parties d'huiles diméthylpolysiloxanes (E) à extrémités silanol de viscosité à 25°C comprise entre 10 et 5000 mPa · s, de préférence de 30 à 1000 mPa · s, pour 100 parties de gomme (A). Leur utilisation est surtout recommandée lorsque les quantités de charges renforçantes (B$_1$) sont élevées.

La préparation des compositions conformes à l'invention s'effectue à l'aide de moyens mécaniques connus, par exemple de pétrins, de mélangeurs à cylindres, de mélangeurs à vis.

Les divers constituants sont incorporés dans ces appareils dans un ordre pouvant être quelconque. Il est toutefois recommandé de charger la gomme (A) puis dans l'ordre les charges siliceuses (B$_1$) et le composé d'iode (C), éventuellement l'additif (E) et, en dernier lieu, le composé (D) et (B$_2$).

Les compositions obtenues sont stables au stockage ; par ailleurs, elles se moulent et s'extrudent aisément ce qui permet de réaliser des formes très variées. Celles qui contiennent du péroxyde (B$_2$) sont réticulées par chauffage. La durée du chauffage varie évidemment avec la température, la pression et la nature des réticulants. Elle est généralement de l'ordre de plusieurs minutes vers 150-250°C et de quelques secondes vers 250-350°C.

Les élastomères ainsi formés peuvent être éventuellement post-chauffés ultérieurement, surtout ceux obtenus par moulage pendant une période d'au moins une heure à une température comprise entre 190 et 270°C dans le but d'achever leur réticulation.

Néanmoins, ces élastomères possèdent dès la fin de leur première phase de réticulation, c'est-à-dire avant la phase éventuelle de post-chauffage, des caractéristiques physiques suffisantes pour l'application envisagée.

Les compositions de silicones éventuellement réticulées se présentent avantageusement sous formes de modules (éléments) de formes variées. Pour un module donné on détermine la quantité d'iode et la durée du relargage.

On peut ainsi immerger un nombre approprié de modules dans les eaux à traiter qui correspond à une quantité (un poids d'élastomère) telle que l'élastomère assure une libération continue et contrôlée d'iode, de préférence pendant au moins un an. Au bout de cette période les modules (éléments) sont remplacés.

De façon surprenante on a découvert que ces compositions de silicones, éventuellement réticulées, présentent des caractéristiques suffisantes pour les applications envisagées.

Les exemples ci-après illustrent l'invention sans en limiter sa portée.

## Exemple 1.

### Préparation de la composition.

Composition : on mélange intimement à l'aide d'un malaxeur les ingrédients suivants :
* 100 parties d'une gomme (A) diméthylméthylvinylpolysiloxane bloquée à chacune de ses deux extrémités par un motif triméthylsiloxy et comprenant dans sa chaîne 99,8% molaire de motif diméthylsiloxy et 0,2% molaire de motifs vinylméthylsiloxy et de viscosité 10 millions de mPa · s à 25°C.
* 43,5 parties de charge (B) qui est une silice de combustion traité D4 (octaméthylcyclotetrasiloxane) de surface spécifique BET de 300 m$^2$/g.
* 1 partie d'un diméthylpolysiloxane linéaire bloqué à ses deux extrémités par des groupements diméthylhydroxysiloxane de viscosité 50 mPa · s.
* 0,2 partie d'octométhyltétracyclosiloxane).
* 37,2 parties de NaI de granulométrie comprise entre 200 et 400 µm.

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :
* 0,5 parties de diméthyl-2,5 di(tertiobutyle peroxy-2,5)hexane.

La composition catalysée se détache aisément des cylindres du mélangeur. Elle est ensuite extrudée en un boudin continu de 26 mm de diamètre qui est ensuite durci par passage dans un four de 6 m porté à 350°C à la vitesse de 1 m par minute. On obtient un jonc uniforme qui est ensuite recuit à 150°C pendant 4 heures.

### Protocole expérimental de la mesure de la cinétique d'élution.

La composition élastomère contenant le NaI est découpé à la longueur désirée (35,4 mm), conformément au rapport Surface/Volume (2,08 cm$^{-1}$) que l'on désire atteindre et immergée dans un récipient de 500 ml d'eau distillée, thermostatée à 20°C.

Le récipient est équipé d'un système d'agitation magnétique, animé d'un mouvement de rotation lent (100 tr/min) assurant l'homogénéité de la solution. Il est recouvert d'un couvercle afin de minimiser l'évaporation de l'eau.

Des prélèvements journaliers de 1 ml sont réalisés dans les premiers temps de l'élution, et hebdomadaires au bout de 15 jours d'élution.

La concentration en iodure ou iodate, libérée par jour, est déterminée par un dosage avec une électrode spécifique à iodure :

A un millilitre de prélèvement du récipient on rajoute deux millilitres d'une solution (K2SO4 + acide ascorbique) – cette solution joue le rôle de tampon ionique, et de solution réductrice dans le cas du dosage des iodates – et un millilitre d'eau distillée. On immerge l'électrode dans cette solution et on lit le potentiel électrochimique de la solution. Un courbe d'étalonnage préalablement établie avec des solutions en iodure de 5.10-5 M/l (M : Mole) à 5.10-2 M/l permet de calculer la concentration (C) en iodure ou iodate en mg/l de la solution.

Les caractéristiques du cylindre immergé sont :
* Diamètre : 26,3 mm
* Hauteur : 35,4 mm
* Surface : 40,1 cm$^2$
* Volume : 19,28 cm$^3$
* S/V : 2,08 cm$^{-1}$
* Masse totale : 19,89 g
* Quantité initiale de I(Qo) : 3,36 g

Dans le tableau 1 sont rassemblés les résultats de la

Tableau 1

| TEMPS (JOUR) | Q cumulé (gramme) ion acti | 100*Q/Q0 % |
|---|---|---|
| 0.19 | 0.189 | 5.60 |
| 0.98 | 0.297 | 8.81 |
| 2.01 | 0.341 | 10.13 |
| 6.92 | 0.355 | 10.53 |
| 11.02 | 0.358 | 10.62 |
| 15.92 | 0.428 | 12.70 |
| 17.92 | 0.440 | 13.08 |
| 24.97 | 0.431 | 12.79 |
| 35.20 | 0.432 | 12.83 |
| 45.91 | 0.535 | 15.89 |
| 52.23 | 0.468 | 13.89 |
| 60.19 | 0.522 | 15.50 |
| 73.21 | 0.622 | 18.45 |
| 77.12 | 0.622 | 18.47 |
| 91.12 | 0.602 | 17.86 |
| 115.92 | 0.712 | 21.13 |
| 150.18 | 0.726 | 21.56 |

cinétique d'élution.

Q correspond à la quantité d'équivalent I (que nous appelons ion actif) éluée à l'instant t.

Sachant que 80% de l'ion actif incorporé élue selon une cinétique d'ordre zéro par rapport au temps, nous pouvons calculer pour chaque exemple le Temps d'élution théorique (Te) selon :

$$Te = \frac{0,8 \times Q_0}{Flux\ journalier} \quad (Jours)$$

Dans le cas de cet exemple le Te est de 640 jours.

### Exemple 2.

On utilise la même composition qu'à l'exemple 1 sauf que l'on incorpore 37,2 parties de $KIO_3$ de granulométrie comprise entre 100 et 200 $\mu$m.

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :

* 0,5 parties de diméthyl-2,5 di(tertiobutyle peroxy-2,5)hexane.

La composition catalysée se détache aisément des cylindres du mélangeur. Elle est ensuite extrudée en un boudin continu de 23 mm de diamètre qui est ensuite durci par passage dans un four de 6 m porté à 350°C à la vitesse de 1 m par minute. On obtient un jonc uniforme qui est ensuite recuit à 150°C pendant 4 heures.

Le protocole de suivi de la cinétique d'élution est identique à celui de l'exemple 1.

On immerge dans 500 ml d'eau distillée thermostatée 20°C, un cylindre dont les caractéristiques sont les suivantes.

* Diamètre : 23,0 mm

TABLEAU 2

| TEMPS (JOUR) | Q cumulé (gramme) ion acti | 100*Q/Q0 % |
|---|---|---|
| 0.12 | 0.009 | 0.31 |
| 0.29 | 0.010 | 0.34 |
| 1.00 | 0.018 | 0.58 |
| 2.00 | 0.022 | 0.71 |
| 6.00 | 0.035 | 1.15 |
| 8.00 | 0.045 | 1.45 |
| 12.00 | 0.051 | 1.66 |
| 15.00 | 0.053 | 1.74 |
| 21.00 | 0.065 | 2.12 |
| 26.00 | 0.071 | 2.32 |
| 33.00 | 0.082 | 2.67 |
| 40.00 | 0.091 | 2.98 |
| 49.00 | 0.104 | 3.41 |
| 61.00 | 0.116 | 3.78 |
| 79.00 | 0.141 | 4.60 |
| 96.00 | 0.161 | 5.24 |
| 117.00 | 0.182 | 5.92 |

* Hauteur : 50,0 mm
* Surface : 44,4 cm$^2$
* Volume : 20,76 cm$^3$
* S/V : 2,14 cm$^{-1}$
* Masse totale : 25,37 g
* Quantité initiale de I(Qo) : 3,01 g

Dans le tableau 2 sont rassemblés les résultats de la cinétique d'élution.
Le temps d'élution théorique de cet exemple est de 1170 jours.

## Revendications

1. Composition de silicone vulcanisable à chaud comportant :
A – une gomme diorganopolysiloxane dont la viscosité à 25°C est supérieure à 1000000 mPa · s,

B$_2$ – un péroxyde organique,

B$_1$ – éventuellement une charge renforçante

C – au moins un composé de l'iode à l'exception de l'iode moléculaire I$_2$, solide à la température ambiante, soluble dans l'eau, non toxique, dispersé de façon homogène dans la composition.

2. Composition selon la revendication 1, caractérisée en ce que pour 100 parties, en poids, de gomme A elle comprend de 5 à 150 parties du composé de l'iode (C).

3. Composition de silicone selon la revendication 1, caractérisée en ce que le composé (B$_1$) est une charge renforçante siliceuse.

4. Composition de silicone selon la revendication 1, caractérisée en ce que le composé de l'iode (C) est un iodure ou iodate de formules générales :

$$(M^{a+}) (I^-)_a$$

et $(M^{a+}) (IO^-_3)_a$

dans lesquelles a est un nombre entier supérieur ou égal à 1 et M est un cation choisi parmi un métal alcalin, un métal alcalino-terreux, un métal de transition et un ammonium quaternaire $(NY_4)^+$ dont les radicaux Y identiques ou différents représentent un radical alkyle linéaire ou ramifié en C$_1$-C$_{20}$ ou un atome d'hydrogène.

5. Composition selon la revendication 1, caractérisée en ce que le composé de l'iode (C) est choisi parmi NaI, NaIO$_3$,

KI, KIO$_3$,

MgI$_2$, MgI$_2 \cdot$ 8H$_2$O,

Mg(IO$_3$)$_2 \cdot$ 4H$_2$O,

NH$_4$I

FeI$_2 \cdot$ 4H$_2$O

MnI$_2$.

6. Composition selon l'une quelconque des revendications précédentes, caractérisée en ce qu'elle comporte :

— (A) : 100 parties en poids d'une gomme diorganopolysiloxane ayant une viscosité supérieure à 1 Million mPa · s à 25°C,

— (B$_1$) : 5 à 130 parties en poids d'une charge siliceuse renforçante choisie parmi les silices de pyrogénation et les silices de précipitation,

— (B$_2$) : 0,1 à 6 parties d'un péroxyde organique,

— (C) : 5 à 150 parties en poids d'un composé de l'iode défini à la revendication 1.

7. Composition selon l'une quelconque des revendications précédentes, caractérisée en ce que la gomme (A) a pour formule générale R$_{3-a}$(R'O)$_a$SiO(R$_2$SiO)$_n$Si(OR')$_a$R$_{3-a}$ dans laquelle les symboles R, identiques ou différents, représentent des radicaux hydrocarbonés en C$_1$-C$_8$, substitués ou non par des atomes d'halogène, des radicaux cyano ; le symbole R' représente un atome d'hydrogène, un radical alkyle en C$_1$-C$_4$, le symbole a représente zéro ou un, le symbole n représente un nombre ayant une valeur suffisante pour obtenir une viscosité d'au moins 1 Million de mPa · s à 25°C, au moins 50% en nombre de radicaux représentés par R sont des radicaux méthyle.

8. Composition selon la revendication 7, caractérisée en ce que 0,005 à 0,5 mole % des motifs entrant dans la constitution de la gomme (A) sont choisis parmi ceux de formules (CH$_2$=CH)(R)SiO et (CH$_2$=CH)R$_{2-a}$(RO')$_a$Si$_{0,5}$.

9. Composition selon l'une quelconque des revendications 1 à 8, caractérisée en ce qu'elle comporte en outre de 0,1 à 6 parties d'un agent de texturation (D) qui est un polymère organofluoré sous forme d'un solide pulvérulent, pour 100 parties de gomme A.

10. Composition selon la revendication 3, caractérisée en ce que jusqu'à 90% en poids de la charge siliceuse renforçante (B$_1$) est remplacée par des charges semi-renforçantes ou de bourrage.

11. Composition selon la revendication 1, durcie en un élastomère par chauffage.

12. Procédé de traitement d'eaux, caractérisé en ce qu'on immerge une quantité adaptée d'une composition durcie telle que définie à la revendication 11 en vue de libérer dans l'eau une quantité continue et contrôlée du composé de l'iode.

EP 0 285 525 B1

**Patentansprüche**

1. In der Wärme vulkanisierbare Siliconzusammensetzung, enthaltend

A – ein Diorganopolysiloxan-Gummi, dessen Viskosität bei 25°C höher als 1000000 mPa · s ist,

$B_2$ – ein organisches Peroxid,

$B_1$ – gegebenenfalls einen verstärkenden Füllstoff,

C – zumindest eine Jodverbindung mit Ausnahme von molekularem Jod $J_2$, die bei Raumtemperatur fest, in Wasser löslich, nicht-toxisch, in der Zusammensetzung homogen dispergiert ist.

2. Zusammensetzung gemäß Anspruch 1, dadurch gekennzeichnet, daß sie je 100 Teile, bezogen auf das Gewicht, des Gummis A 5 bis 150 Teile Jodverbindung (c) enthält.

3. Siliconzusammensetzung gemäß Anspruch 1, dadurch gekennzeichnet, daß die Verbindung ($B_1$) ein siliciumhaltiger, verstärkender Füllstoff ist.

4. Siliconzusammensetzung gemäß Anspruch 1, dadurch gekennzeichnet, daß die Jodverbindung (C) ein Jodid oder Jodat der allgemeinen Formeln

$(M^{a+}) (J^-)_a$       und

$(M^{a+}) (JO_3^-)_a$

ist, worin a eine ganze Zahl von höher als oder gleich 1 ist und M ein Kation ist, ausgewählt unter einem Alkalimetall, einem Erdalkalimetall, einem Übergangsmetall und einem quaternären Ammonium $(NY_4)^+$, dessen Reste Y, die gleich oder verschieden sind, einen linearen oder verzweigten $C_{1-20}$-Alkylrest oder ein Wasserstoffatom bedeuten.

5. Zusammensetzung gemäß Anspruch 1, dadurch gekennzeichnet, daß die Jodverbindung (C) ausgewählt ist unter :

NaJ, $NaJO_3$,

KJ, $KJO_3$,

$MgI_2$, $MgJ_2 · 8H_2O$,

$Mg(JO_3)_2 · 4H_2O$,

$NH_4J$

$FeJ_2 · 4H_2O$

$MnJ_2$.

6. Zusammensetzung gemäß einem der vorhergehenden Ansprüche, dadurch gekennzeichnet, daß sie enthält :

(A) 100 Gew.Teile eines Diorganopolysiloxan-Gummis mit einer Viskosität von höher als 1 Million mPa·s bei 25°C,

($B_1$) 5 bis 130 Gew.Teile eines verstärkenden, siliciumhaltigen Füllstoffs, ausgewählt unter den Pyrolyse-Siliciumdioxiden und den Ausfällungs-Siliciumdioxiden,

($B_2$) 0,1 bis 6 Teile eines organischen Peroxids,

(C) 5 bis 150 Gew.Teile einer in Anspruch 1 definierten Jodverbindung.

7. Zusammensetzung gemäß einem der vorhergehenden Ansprüche, dadurch gekennzeichnet, daß das Gummi (A) die allgemeine Formel $R_{3-a}(R'O)_aSiO(R_2SiO)_nSi(OR')_aR_{3-a}$ besitzt, worin die Symbole R, die gleich oder verschieden sind, $C_{1-8}$-Kohlenwasserstoffreste, welche substituiert oder unsubstituiert sind durch Halogenatome, Cyanogruppen, wiedergeben ; das Symbol R' ein Wasserstoffatom, einen $C_{1-4}$-Alkylrest bedeutet; das Symbol a für 0 oder 1 steht ; das Symbol n eine Zahl mit einem ausreichenden Wert wiedergibt, um eine Viskosität von zumindest 1 Million mPa · s bei 25°C zu erreichen, wobei zumindest 50% der Anzahl der durch R dargestellten Reste Methylreste sind.

8. Zusammensetzung gemäß Anspruch 7, dadurch gekennzeichnet, daß 0,005 bis 0,5 Mol-% der in die Konstitution des Gummis (A) eintretenden Gruppen unter denjenigen der Formeln $(CH_2=CH)(R)SiO$ und $(CH_2=CH)R_{2-a}(RO')_aSi_{0,5}$ ausgewählt sind.

9. Zusammensetzung gemäß einem der Ansprüche 1 bis 8, dadurch gekennzeichnet, daß sie außerdem 0,1 bis 6 Teile eines Texturierungsmittels (D), das ein Organofluorpolymeres in Form eines pulverförmigen Feststoffs ist, je 100 Teile Gummi A enthält.

10. Zusammensetzung gemäß Anspruch 3, dadurch gekennzeichnet, daß bis zu 90 Gew.% des verstärkenden, siliciumhaltigen Füllstoffs ($B_1$) durch halbverstärkende Füllstoffe oder Füllmaterialien ersetzt sind.

11. Zusammensetzung gemäß Anspruch 1, durch Erhitzen zu einem Elastomeren gehärtet.

12. Verfahren zur Behandlung von Wässer, dadurch gekennzeichnet, daß man eine geeignete Menge einer gehärteten Zusammensetzung, wie in Anspruch 11 definiert, im Hinblick darauf eintaucht, daß in Wasser eine kontinuierliche und Kontrollierte Menge Jodverbindung freigesetzt wird.

13

## Claims

1. Hot-vulcanisable silicone composition comprising :

A – a diorganopolysiloxane gum, the viscosity of which at 25°C is higher than 1,000,000 mPa · s,

$B_2$ – an organic peroxide,

$B_1$ – if appropriate, a reinforcing filler,

C – at least one iodine compound, with the exception of molecular iodine $I_2$, which is solid at the ambient temperature, soluble in water, non-toxic and dispersed homogeneously in the composition.

2. Composition according to Claim 1, characterised in that per 100 parts by weight of gum A it comprises from 5 to 150 parts of the iodine compound (C).

3. Silicone composition according to Claim 1, characterised in that the compound $(B_1)$ is a silicious reinforcing filler.

4. Silicone composition according to Claim 1, characterised in that the iodine compound (C) is an iodide or iodate of general formulae :

$(M^{a+}) (I^-)_a$

and

$(M^{a+}) (IO_3^-)_a$

in which formulae a is an integer greater than or equal to 1 and M is a cation chosen from an alkali metal, an alkaline earth metal, a transition metal and a quaternary Ammonium $(NY_4)^+$ in which the radicals Y, which may be identical or different, represent a $C_1$-$C_{20}$ straight-chain or branched alkyl radical or a hydrogen atom.

5. Composition according to Claim 1, characterised in that the iodine compound (C) is chosen from

$NaI$, $NaIO_3$,

$KI$, $KIO_3$,

$MgI_2$, $MgI_2 \cdot 8H_2O$,

$Mg(IO_3)_2 \cdot 4H_2O$,

$NH_4I$,

$FeI_2 \cdot 4H_2O$ and

$MnI_2$.

6. Composition according to any one of the preceding claims, characterised in that it comprises :

— (A) :     100 parts by weight of a diorganopolysiloxane gum having a viscosity higher than 1 million mPa · s at 25°C,

— $(B_1)$ :     5 to 130 parts by weight of a reinforcing silicious filler chosen from silica produced by pyrolysis and silica produced by precipitation,

— $(B_2)$ :     0.1 to 6 parts of an organic peroxide, and

— (C) :     5 to 150 parts by weight of an iodine compound defined in claim 1.

7. Composition according to any one of the preceding claims, characterised in that the gum (A) has the general formula $R_{3-a}(R'O)_aSiO(R_2SiO)_nSi(OR')_aR_{3-a}$, in which the symbols R, which may be identical or different, represent $C_1$-$C_8$ hydrocarbon radicals, which are unsubstituted or substituted by halogen atoms or cyano radicals ; the symbol R' represents a hydrogen atom or a $C_1$-$C_4$ alkyl radical, the symbol a represents zero or one and the symbol n represents a number having a value sufficient to obtain a viscosity of at least 1 million mPa·s at 25°C, and at least 50% by number of the radicals represented by R are methyl radicals.

8. Composition according to Claim 7, characterised in that 0.005 to 0.5 mol-% of the units present in the structure of the gum (A) are chosen from those of formulae $(CH_2=CH)(R)SiO$ and $(CH_2=CH)R_{2-a}(RO')_aSi_{0.5}$.

9. Composition according to any one of Claims 1 to 8, characterised in that it also comprises from 0.1 to 6 parts of a structuring agent (D), which is an organofluorinated polymer in the form of a pulverulent solid, per 100 parts of gum A.

10. Composition according to Claim 3, characterised in that up to 90% by weight of the reinforcing silicious filler $(B_1)$ is replaced by semi-reinforcing or packing fillers.

11. Composition according to Claim 1, cured to form an elastomer by heating.

12. Process for water treatment, characterised in that a suitable amount of a cured composition as defined in Claim 11 is immersed, with a view to liberating a continuous and controlled amount of the iodine compound into the water.